# EUROPEAN PATENT SPECIFICATION

(11) **EP 1 520 545 B1**
(45) Date of publication and mention of the grant of the patent: **29.11.2006**
(21) Application number: 03292425.0
(22) Date of filing: 01.10.2003
(51) Int. Cl.: A61B 17/70, A61B 17/80, A61F 2/44

(54) **Spinal device locking mechanism**
Verriegelungseinrichtung für eine Wirbelsäulen-Fixationsvorrichtung
Mécanisme de blocage pour système de fixation rachidien

(43) Date of publication of application: 06.04.2005
(73) Proprietor: Warsaw Orthopedic, Inc., Warsaw, IN 46581 (US)
(72) Inventor: Dupont, Philippe, 77410 Claye-Souilly (FR); D'Amore, Jean-François, 77144 Montevrain (FR)
(74) Representative: Neyret, Daniel Jean Marie

(56) References cited:
- US-A- 6 139 550
- US-A1- 2002 052 656
- US-A1- 2002 147 450
- US-A1- 2003 040 749
- US-B1- 6 306 139
- US-B1- 6 540 785

## Description

### FIELD OF THE INVENTION

The present invention relates generally to spinal systems, and more particularly, to a spinal or other orthopedic device with a screw locking mechanism that prevents screws from pulling out of the device.

### BACKGROUND

The treatment of injuries to the spine has advanced significantly, including treatment for many forms of spinal injuries and deformities that can occur due to disease, congenital effects, the effects of tumors, and, of course, fractures and dislocations attributable to physical trauma. For many years, the use of plates secured to the spine by screws has been helpful in the stabilization and fixation of the spine. Such plate and screw systems have not been limited to treating spinal injuries, but have also been used in the treatment of other bone injuries.

An important recognized requirement for a plating system is that the screws used to connect the plate or other device to the vertebrae or other bone mass must not loosen or back out from the plate over time.

US-A-6 139 550 and US-B1-6 540 785 disclose plating systems build according to the preambles of claims 1 and 15 of the present patent.

What is needed is a locking mechanism that is simple to fabricate, does not have a threaded surface and can be used with many different materials.

### SUMMARY

The present invention provides a new and improved locking mechanism and spinal orthopedic device. In one embodiment, an improved spinal orthopedic device includes a key and a plate. The key includes a cover, a post and a heel, the post having a first end connected to the cover and a second end connected to the heel. The plate includes a heel receiving hole and a mechanical construct for engaging with an attachment mechanism, such as a bone screw hole for engaging with a bone screw. The key and plate are configured so that the heel is insertable through the heel receiving hole and is rotatable when inserted in the heel receiving hole. In addition, the cover is shaped so that when rotated, the cover overlaps at least a portion of the mechanical construct to thereby prevent disengagement of the attachment mechanism.

In another embodiment, an improved spinal device includes a plate including a heel receiving hole and a plurality of screw holes for engaging with a plurality of screws, and a locking mechanism connected to the plate. The plurality of screw holes are configured so that the plurality of screws may be inserted there through and may be further connected to a vertebrae of the spine, thereby securing the plate to the vertebrae. The locking mechanism includes a key having a cover connected to a post, further connected to a heel. The heel is shaped so that it is insertable through the heel receiving hole in one configuration, is rotatable when inserted, and is secured to the plate after being rotated. The cover is configurable so that when the heel is secured to the plate, the cover engages, at least in part, with the plurality of screws when the plate is secured to the vertebrae via the plurality of screws.

In another embodiment, an improved intervertebral joint prosthesis includes a ball component for engagement with a first vertebra, a trough component for engagement with a second vertebra, and first and second locking mechanisms. The ball component includes a generally convex surface, and a first flange plate at one end of said ball component for engaging the first vertebra. The first flange plate includes a first locking mechanism opening for engaging with the first locking mechanism and a first bone attachment opening for engaging with a first bone attachment mechanism. The trough component includes a generally concave surface, and a second flange plate at one end of said trough component for engaging the second vertebra. The second flange plate includes a second locking mechanism opening for engaging with the second locking mechanism and a second bone attachment opening for engaging with a second bone attachment mechanism. Each of the first and second locking mechanisms includes a cover connected to post further connected to a heel. The heel is configured so as to be insertable into and through the corresponding heel plate opening. The post being is configured so as to be rotatable within the heel plate opening after the heel is inserted there through. The cover is configured to secure an engaged bone attachment mechanism when the post is rotated within the heel plate.

Many objects and benefits of the invention will become apparent upon consideration of the following written description of the invention, together with the accompanying Figures.

### BRIEF DESCRIPTION OF THE DRAWINGS

Figs. 1a-1c are front and cross-sectional views of an anterior cervical plate system implementing features according to one embodiment of the present invention.

Figs. 2a-2d are perspective and cross-sectional views of an intervertebral joint prosthesis implementing features according to another embodiment of the present invention.

Figs 3a-3c are side, bottom, and cross-sectional views-of the key portion of the screw locking mechanism according to one embodiment of the present invention.

Fig. 4 is a top perspective view of the plate portion of the screw locking mechanism according to one embodiment of the present invention.

Fig. 5 is a side cross-sectional view of the plate shown in Fig. 4.

Fig. 6 is a top perspective view of the plate shown in Fig. 4 with the key shown in Fig. 1 engaged.

Fig. 7 is a cross-sectional view of the plate with engaged key as shown in Fig. 6.

Fig. 8 is a side elevational view of the key portion of the screw locking mechanism according to another embodiment of the present invention.

Fig. 9 is a bottom perspective view of the key shown in Fig. 8.

### DETAILED DESCRIPTION

For the purposes of promoting an understanding of the principles of the invention, reference will now be made to the embodiments, or examples, illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates.

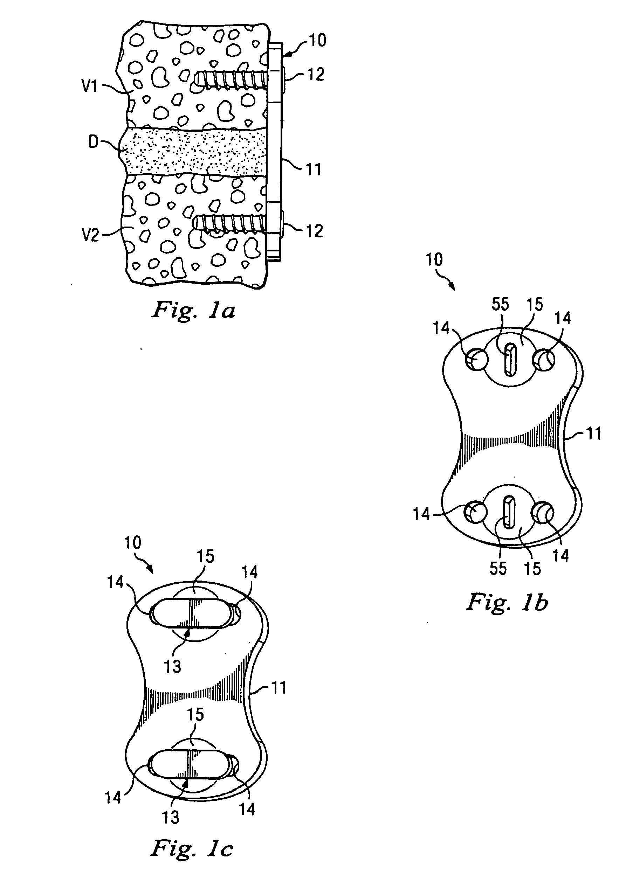

Referring to Figs. 1a, 1b, and 1c, an anterior cervical plate system 10 is an example of an orthopedic implant that can benefit from different embodiments of the present invention. In accordance with the present example, the plating system 10 includes an elongated plate 11 and one or more bone attachment mechanisms 12, such as bone screws, attached to adjacent vertebrae V1, V2. Other bone attachment mechanisms include bolts, staples, and protrusions that can help to secure the plate in a desired position, and are hereinafter generically referred to as bone screws. The bone screws 12 are held or retained to the plate 11 by way of one or more locking mechanisms 13, discussed in greater detail below. The elongated plate 11 is provided with a plurality of openings or holes 14 in a variety of arrangements. Further, the plate 11 includes recesses 15 between two or more of the holes 14.

The plate 11 and/or locking mechanisms 13 can be constructed of many different materials, including metal materials such as titanium or radiolucent material, such as a polymer based resin. An example of a plate 11 constructed substantially from a polyether ether ketone (PEEK) high temperature thermoplastic is discussed in greater detail in U.S. A-2004/0215195.

The plate 11 can further include one or more bone growth or fusion-promoting elements, such as bone, bone morphogenetic protein (BMP), demineralized bone matrix (DBM), LIM mineralization proteins (LMP), osteogenic pastes, and so forth. It is understood that such fusion-promoting elements are well known by those of ordinary skill in the art.

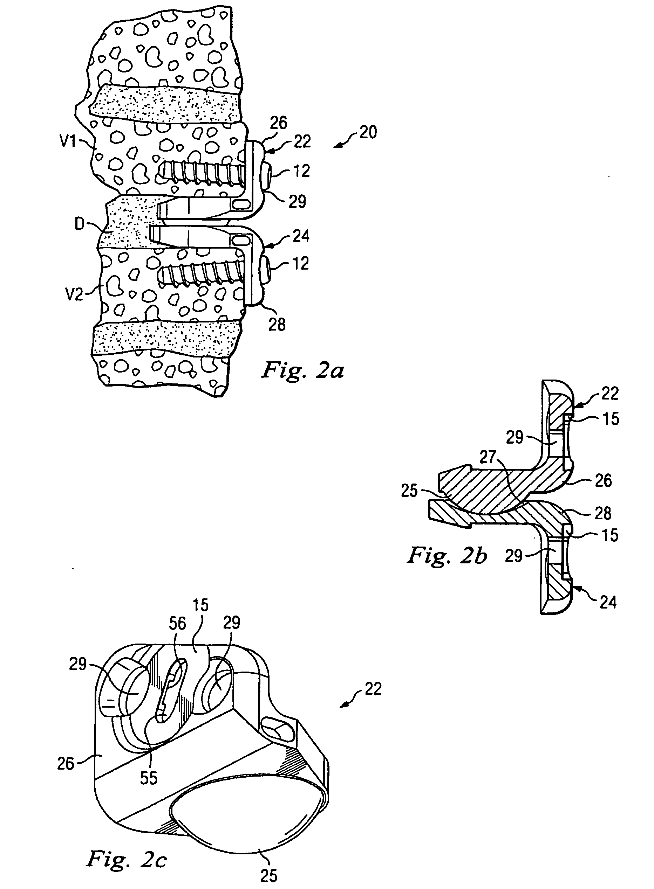

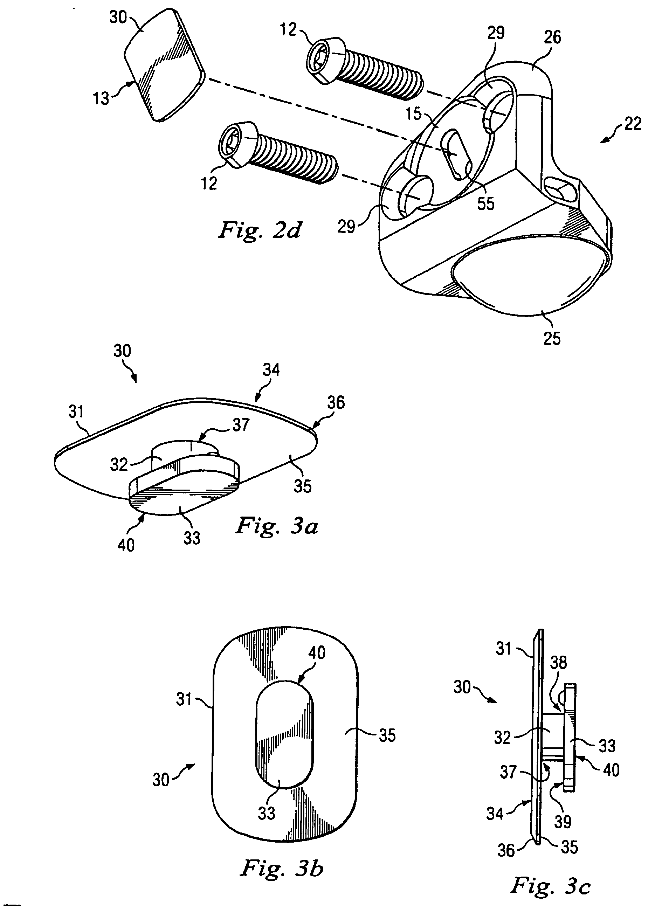

Referring to Figs. 2a-2d, an intervertebral joint prosthesis system 20 is another example of an orthopedic implant that can benefit from different embodiments of the present invention. In accordance with the present example, the prosthesis 20 includes a ball component 22 and a trough component 24 that are inter-engageable to form the prosthesis. In an intervertebral disc space D between two adjacent vertebrae V1, V2, ball component 22 is fixed to one of the adjacent vertebrae (e.g. vertebra V1), and trough component 24 is fixed to the other adjacent vertebra (e.g. vertebra V2) so that the components are inter-engaged within at least a portion of intervertebral space D.

Ball component 22 includes a generally convex surface 25. In a particular embodiment, generally convex surface 25 is substantially hemispherical in shape. A flange plate 26 is provided at one end of the ball component 22 for attaching the ball component to a vertebra, and is preferably formed to have a low profile. Trough component 24 includes a generally concave surface 27. A flange plate 28 is provided at one end of the trough component 24 for attaching the trough component to a vertebra, and is preferably formed to have a low profile. The ball and trough components 22, 24 are discussed in greater detail in U.S. Patent No. 6,540,785.

In a particular embodiment, flange plates 26 and/or 28 include one or more bone screw apertures 29, and in a specific embodiment, two bone screw apertures are provided in a symmetric relationship through each flange plate. In that specific embodiment, one or more bone screws 12 are threaded into the vertebra through the apertures 29 to fix the components 22, 24 to the vertebra. After the components 22, 24 are attached to the vertebrae using bone screw(s) 12, a locking mechanism 13 is used to secure the screws 12 in place. The locking mechanism 13 includes a locking key 30, both of which are discussed in greater detail, below.

The components 22, 24 and/or locking mechanisms 13 can be constructed of many different materials, including metal materials such as titanium or radiolucent material as discussed above with respect to Figs. 1a-1c. Also, the components 22, 24 can further include one or more bone growth or fusion-promoting elements, such as bone, BMP, DBM, LMP, osteogenic pastes, and so forth.

Referring to Figs. 3a-3c, in one embodiment, the locking mechanism(s) 13 used in the systems 10, 20 discussed above include a locking key 30. In accordance with the present embodiment, the key 30 includes a cover 31, a post 32 and a heal 33. The cover 31 has a top surface 34, a bottom surface 35 and a shoulder 36. The post 32 includes a first end 37, that is secured to the bottom surface 33 of the cover 31, and a second end 38. The heel 33 has a top surface 39, that is secured to the second end 38 of the post 32, and a bottom surface 40. The key 30 can be made from a combination of materials. For example, the heel 33 or the cover 31 may be made of a more deformable material or design, while the post 32 is made of a more rigid material.

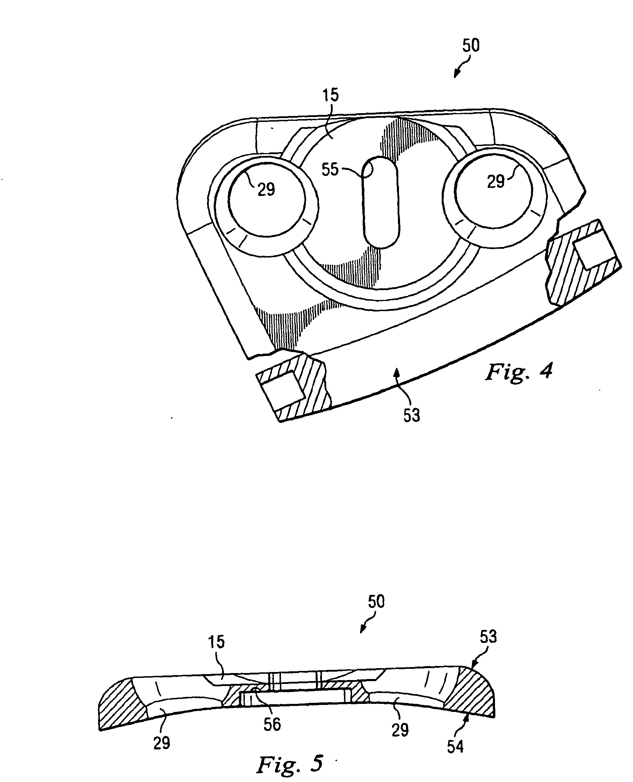

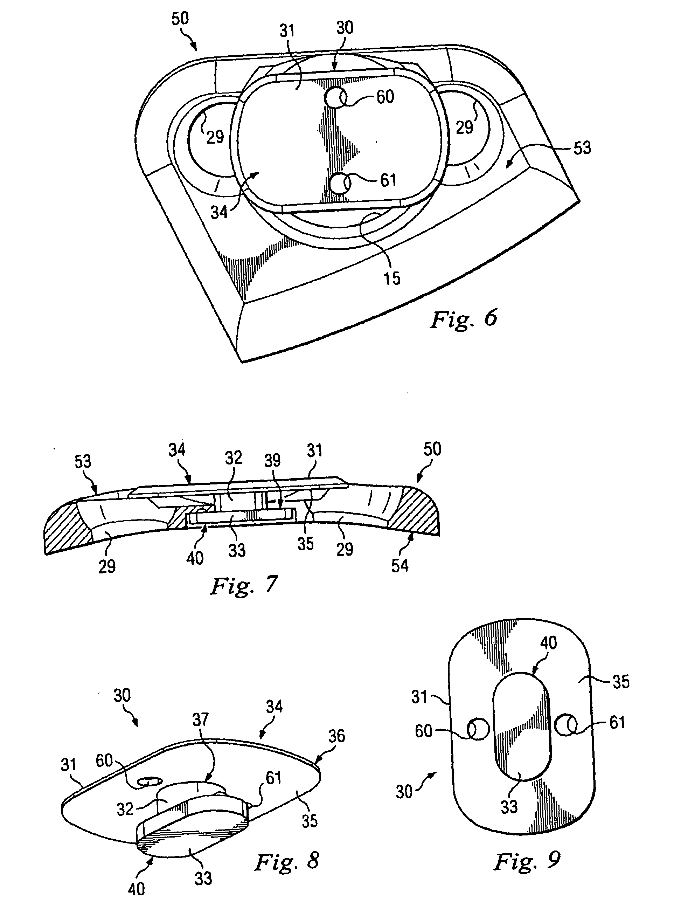

Referring to Figs. 4-7, a plate 50 is representative of the plate 11 of Figs. 1a-1c or the flange plates 26 and/or 28 of Figs. 2a-2d. The plate 50 is designed to receive the heel 33 and post 32 of the key 30, includes one or more screw receiving holes 29, that each permit a screw, such as a spine or other bone screw, to pass through from the top side 53 through the bottom side 54. As shown in Fig. 7, when the key 30 is inserted in the plate 50, as is described below, the bottom surface 35 of the cover 31 covers a portion of the screw receiving holes 29 and engage the heads of screws inserted in the screw receiving holes 29 to prevent the screws from backing out of the plate 50.

The plate 50 also includes a heel receiving hole 55 that is shaped to permit the heel 33 of the key 30 to pass through such that the top surface 39 of the heel 33 extends beyond at least a portion of the bottom side 54 of the plate 50 adjacent to the heel receiving hole 55. A portion of the plate 50 surrounding the heel receiving hole 55 may be of a more deformable material than the remaining portions of the plate. The post 32 and heel receiving hole 55 are each shaped such that when the key 30 is fully inserted in the plate 50 and rotated, the post 32 is permitted to rotate within the heel receiving hole 55.

The heel 33 and heel receiving hole 55 are designed to permit the heel 33 to pass through the heel receiving hole 55, but once the key 30 is rotated, if one attempts to remove the key 30 from the plate 50, the top surface 39 of the heel 33 will engage the bottom side 54 of the plate 50 and prevent the key 30 from pulling out of the plate 50. It is appreciated that in order to provide for this relationship between the heel 33 and the heel receiving hole 55, the heel 33 must have a non-circular shape and at least a portion of the heel 33 must extend out from the diameter of the second end 38 of the post 32.

The bottom side 54 of the plate 50 is constructed with a lodgment 56 that is designed to receive and secure the heal 33, and thus the key 30, relative to the plate 50. In one embodiment, as shown in Figs. 2c, 3a, and 5a, the lodgment includes a corresponding bump and groove in the heal 33 and plate 50. The lodgment 56 engages when the key 30 is rotated within the heel receiving hole 55.

While the post 32 and the heel receiving hole 55 may be designed to allow the post 32 to rotate 360 degrees within the heel receiving hole 55, absent any other restriction created by the lodgment 56, they may also be designed such that the post 32 is only permitted to rotate sufficiently to enable the heal 33 top surface 39 to contact the plate 50 bottom side 54 when the key 30 is attempted to be removed from the plate 50. Additionally, in the present embodiment, the key 30 is only permitted to rotate relative to the plate 50 enough to secure the heel 33 with the lodgment 56.

It is understood that the lodgment need not be placed on the bottom side 54 of the plate 50, but could also be formed in the wall of the heel receiving hole 55 with the post 32 having a counterpart surface that can be secured in the lodgment. In other embodiments, the lodgment can be a wedge, a lip, or some other protrusion to help prevent the key 30 from rotating in the heel receiving hole 55. Further, the lodgment could be formed in the top side 53 of the plate 50 with the bottom surface 35 of the cover 31 having a counterpart surface to secure in the lodgment. In still other embodiments, the lodgment may be a roughened surface 40 of the heel 33 that engages with the corresponding vertebral body V1 or V2 (Figs. 1a, 2a, and 3a).

In order to rotate the key 30 inside the heel receiving hole 55, the cover 31 of the present embodiment is configured to allow for easy rotation. For example, in Figs. 8-9, a modification to the cover 31 is shown with two tool receiving holes 60 and 61 included in the top surface 34. These tool receiving holes 60 and 61 enable a tool to be inserted in the tool receiving holes 60 and 61 to assist with the rotation of the key 30. Similarly, the top surface 34 of the cover 31 could include a channel for receiving the end of a standard flat head screw driver to assist with the rotation of the key 30. Further, the cover 31 itself could be shaped in such a manner that a device could be placed around its circumference, such as a socket wrench, to assist with the rotation of the key 30. It should be appreciated that a number configurations of depressions in or structures affixed to the cover 31, or the shape of the cover 31 itself could be designed to cooperate with a tool, or to be more easily grasped by a hand, that would assist with the rotation of the key 30 to make it easier to rotate relative to the plate 50.

It is understood that while the cover 31 could at all times cover a portion of the screw receiving holes 29 when the key 30 is inserted in the heel receiving hole 55, it is only necessary for proper operation of the present invention that the cover 31 be positioned relative to the heel 33 such that when the heel 33 is secured in the lodgment 56, the cover 31 covers all or a portion of the heads of screws secured in the screw receiving holes 29, thus preventing them from backing out of the plate 50.

Additionally, it is understood that when the key 30 is secured relative to the plate 50, the bottom surface 35 of the cover 31 can smoothly engage the top side 53 of the plate 50, such that a smooth transition exists moving from the top side 53 of the plate 50 over the shoulder 35 to the top surface 34 of the cover.

The screw locking mechanism of the present invention includes many benefits. For example, the key and plate design does not require the use of a threaded surface. Further, the plate only requires a simple heel receiving hole with a non-complex lodgment. Finally, because of the use of deformation and lodgment, the screw locking mechanism of the current invention can be designed in an material that permits sufficient deformation to secure the key relative to the plate.

Although only a few exemplary embodiments of this invention have been described in detail above, those skilled in the art will readily appreciate that many modifications are possible in the exemplary embodiments without materially departing from the novel teachings and advantages of this invention. Also, features illustrated and discussed above with respect to some embodiments can be combined with features illustrated and discussed above with respect to other embodiments. Accordingly, all such modifications are intended to be included within the scope of the invention as defined in the claims.

## Claims

1. A spinal orthopaedic device comprising:
a key (30) having a cover (31), a post (32) and a heel (33), the post (32) having a first end (37) connected to the cover (31) and a second end (38) connected to the heel (33) ; and at least a portion of the heel (33) extending out from the diameter of the second end (38) of the post (32)
a plate (50) having a non-circular heel receiving hole (55), a mechanical construct for engaging with at least one attachment mechanism to a vertebra, the bottom side of the plate (50) being designed to receive and secure the heel (33) once the key (30) is rotated, the key (30) and plate (50) being configured so that the heel is insertable through the heel receiving hole (55) and is rotatable when inserted in the heel receiving hole (55), and
the cover (31) being shaped so that when rotated, the cover (31) overlaps at least a portion of the mechanical construct to thereby prevent disengagement of the attachment mechanism,
**characterized in that** the bottom side of the plate comprises a bottom recess and the bottom recess allows the heel (33) to be contained therein when the key (30) is rotated from an insertion position to an overlapping position so that the heel (33) does not extend beyond the bottom surface of the plate (50).

2. The spinal orthopaedic device of claim 1 wherein the at least one attachment mechanism is a bone screw (12) for engaging with a vertebral body (V1,V2) and the mechanical construct is a bone screw receiving hole (29).

3. The spinal orthopaedic device of claim 1 or 2 wherein the plate (50) has a top side (53) and a bottom side (54), the bottom side (54) having a lodgment (56) proximate the heel receiving hole (55) to enable the heel (33) to rotatably engage the lodgment (56).

4. The spinal orthopaedic device of one of claims 1 to 3, wherein the plate (50) includes a wall that forms the heel receiving hole (55) and a lodgment (56) is formed as a part of the wall, the post (32) having a shape that rotatably engages the lodgment (56).

5. The spinal orthopaedic device of claim 4 wherein the post (32) has a non-circular shape.

6. The spinal orthopaedic device of one of claims 1 to 5 wherein the heel (33) and heel receiving hole (55) are non-circular, such that after inserting the key (30) into the heel receiving hole (55), the rotation of the key (30) relative to the plate (50) positions the heel (33) such that the heel (33) prevents the key (30) from being removed from the heel receiving hole (55).

7. The spinal orthopaedic device of one of claims 1 to 6, wherein the plate (50) includes a recess (15) and the cover (31) flushly engages the plate inside the recess (15).

8. The spinal orthopaedic device of claim 7, wherein the cover (31) has a top surface (34) and a bottom surface (35), such that when the bottom surface (35) engages the plate (50), there is a smooth transition from the plate (50) to the top surface (34) of the cover.

9. The spinal orthopaedic device of one of claims 1 to 8, wherein the cover (31) includes a rotation assist portion (60, 61).

10. The spinal orthopaedic device of one of claims 1 to 9, wherein the plate (50) is sized to span two or more vertebral bodies (V1.V2).

11. The spinal orthopaedic device of claim 1 comprising:
two attachment mechanisms in the form of bone screws (12); and
two mechanical constructs in the form of bone screw receiving holes (14) corresponding to the two bone screws (12),
wherein each bone screw (12) includes a head portion that is larger than the corresponding bone screw receiving hole (14) and a bone attachment portion that is smaller than the corresponding bone screw receiving hole (14); and
wherein the cover (31) is shaped so that when rotated, the cover (31) simultaneously overlaps both bone screw head portions when inserted into their corresponding bone screw receiving holes (14) to thereby prevent disengagement of the bone screws (12).

12. The spinal device of one of claims 1 to 11, wherein the plate (50) is formed of a first material and heel (33) of the locking mechanism is formed of a second material.

13. The spinal device of one of claims 1 to 12, wherein the heel receiving hole (55) has a shape that is non-circular and the heel (33) is of the same shape as the heel receiving hole (55).

14. The spinal device of claim 13, wherein the shape is oval.

15. An intervertebral joint prosthesis, comprising:
a ball component (22) for engagement with a first vertebra (V1), said ball component (22) including a generally convex surface (25), and a first flange plate (26) at one end of said ball component (22) for engaging the first vertebra (V1), the first flange plate (26) including a first locking mechanism opening for engaging with a first locking mechanism and a first bone attachment opening (29) for engaging with a first bone attachment mechanism (12);
a trough component (24) for engagement with a second vertebra (v2) adjacent to the first vertebra (V1), said trough component (24) including a generally concave surface (27), and a second flange plate (28) at one end of said trough component (24) for engaging the second vertebra (V2), the second flange plate (28) including a second looking mechanism opening for engaging with a second locking mechanism and a second bone attachment opening (29) for engaging with a second bone attachment mechanism (12); and
the first and second locking mechanisms for engaging with the first (26) and the second (28) flange plates, respectively, **characterized in that** each of said locking mechanisms includes a key (30) having a cover (31), a post (32) and heel (33), the post (32) having a first end (37) connected to the cover (31) and a second end (38) connected to the heel at least a portion of the heel (33) extending out from the diameter of the second end (38) of the post (32); and
each of said flange plate (26,28) having a non-circular heel receiving hole (55), a mechanical construct for engaging with at least one attachment mechanism to a vertebra (V1,V2) and a bottom recess on a bottom side of the flange plate (26,28), the bottom recess being designed to receive and secure the heel (33) once the key (30) is rotated, the key (30) and flange plate (26,28) being configured so that the heel is insertable through the heel receiving hole (55) and is rotatable when inserted in the heel receiving hole (55), and the cover (31) being shaped so that when rotated, the cover (31) overlaps at least a portion of the mechanical construct to thereby prevent disengagement of the attachment mechanism, and
the bottom recess allows the heel (33) to be contained therein when the key (30) is rotated from an insertion position to an overlapping position so that the heel (33) does not extend beyond the bottom surface of the flange plate (26,28).

16. The intervertebral joint prosthesis of claim 15 wherein the first flange plate (26) includes two bone attachment mechanism openings (29) for engaging with two different bone attachment mechanisms (12), and wherein the first locking mechanism cover (30) is configured to secure both of the bone attachment mechanisms (12) of the first flange plate (26).

17. The intervertebral joint prosthesis of claim 15 or 16 wherein the first flange plate (26) includes a lodgement (56) for preventing the first locking mechanism from further rotation after the first locking mechanism has been rotated to a position in which the cover (30) secures the first bone attachment mechanism (12) in place.

## Patentansprüche

1. Wirbelsäulenorthopädische Vorrichtung, aufweisend:
einen Keil (30) mit einer Abdeckung (31), einem Zapfen (32) und einem Ansatz (33), wobei der Zapfen (32) ein erstes Ende (37), das mit der Abdeckung (31) verbunden ist, und ein zweites Ende (38) aufweist, das mit dem Ansatz (33) verbunden ist, und wobei sich wenigstens ein Teil des Ansatzes (33) von dem Durchmesser des zweiten Endes (38) des Zapfens (32) weg erstreckt,
eine Platte (50) mit einem nichtkreisförmigen Ansatzaufnahmeloch (55) und einer mechanischen Konstruktion für den Eingriff mit wenigstens einem Befestigungsmechanismus an einem Wirbel, wobei die untere Seite der Platte (50) derart gestaltet ist, dass sie den Ansatz (33) aufnimmt und sichert, sobald der Keil (30) gedreht wird, wobei der Keil (30) und die Platte (50) derart konfiguriert sind, dass der Ansatz durch das Ansatzaufnahmeloch (55) hindurch einsetzbar ist und drehbar ist, wenn er in dem Ansatzaufnahmeloch (55) eingesetzt ist, und
wobei die Abdeckung (31) dergestalt ist, dass, wenn sie gedreht ist, die Abdeckung (31) wenigstens einen Teil der mechanischen Konstruktion überlappt, um **dadurch** ein Lösen des Befestigungsmechanismus zu verhindern,
**dadurch gekennzeichnet, dass** die untere Seite der Platte eine untere Ausnehmung aufweist und die untere Ausnehmung ermöglicht, dass der Ansatz (33) in dieser aufgenommen wird, wenn der Keil (30) von einer Einsetzposition in eine Überlappungsposition derart gedreht ist, dass sich der Ansatz (33) nicht über die untere Fläche der Platte (50) hinaus erstreckt.

2. Wirbelsäulenorthopädische Vorrichtung nach Anspruch 1, wobei der wenigstens eine Befestigungsmechanismus eine Knochenschraube (12) für den Eingriff mit einem Wirbelkörper (V1, V2) ist, und die mechanische Konstruktion ein Knochenschraubenaufnahmeloch (29) ist.

3. Wirbelsäulenorthopädische Vorrichtung nach Anspruch 1 oder 2, wobei die Platte (50) eine obere Seite (53) und eine untere Seite (54) aufweist, wobei die untere Seite (54) eine Aushöhlung (56) benachbart zu dem Ansatzaufnahmeloch (55) aufweist, um zu ermöglichen, dass der Ansatz (33) mit der Aushöhlung (56) drehbar in Eingriff gelangt.

4. Wirbelsäulenorthopädische Vorrichtung nach einem der Ansprüche 1 bis 3, wobei die Platte (50) eine Wand aufweist, die das Ansatzaufnahmeloch (55) bildet, und eine Aushöhlung (56) als ein Teil der Wand ausgebildet ist, wobei der Zapfen (32) eine Form hat, die mit der Aushöhlung (56) drehbar in Eingriff gelangt.

5. Wirbelsäulenorthopädische Vorrichtung nach Anspruch 4, wobei der Zapfen (32) eine nichtkreisförmige Gestalt hat.

6. Wirbelsäulenorthopädische Vorrichtung nach einem der Ansprüche 1 bis 5, wobei der Ansatz (33) und das Ansatzaufnahmeloch (55) nichtkreisförmig sind, so dass nach dem Einsetzen des Keils (30) in das Ansatzaufnahmeloch (55) die Drehung des Keils (30) relativ zu der Platte (50) den Ansatz (33) derart positioniert, dass der Ansatz (33) verhindert, dass der Keil (30) aus dem Ansatzaufnahmeloch (55) entfernt wird.

7. Wirbelsäulenorthopädische Vorrichtung nach einem der Ansprüche 1 bis 6, wobei die Platte (50) eine Ausnehmung (15) aufweist, und die Abdeckung (31) bündig mit der Platte innerhalb der Ausnehmung (15) in Eingriff steht.

8. Wirbelsäulenorthopädische Vorrichtung nach Anspruch 7, wobei die Abdeckung (31) eine obere Fläche (34) und eine untere Fläche (35) derart aufweist, dass, wenn die untere Fläche (35) mit der Platte (50) in Eingriff steht, ein sanfter Übergang von der Platte (50) zu der oberen Fläche (34) der Abdeckung vorhanden ist.

9. Wirbelsäulenorthopädische Vorrichtung nach einem der Ansprüche 1 bis 8, wobei die Abdeckung (31) einen die Drehung unterstützenden Abschnitt (60, 61) aufweist.

10. Wirbelsäulenorthopädische Vorrichtung nach einem der Ansprüche 1 bis 9, wobei die Platte (50) derart bemessen ist, dass sie zwei oder mehrere Wirbelkörper (V1, V2) überspannt.

11. Wirbelsäulenorthopädische Vorrichtung nach Anspruch 1, aufweisend:
zwei Befestigungsmechanismen in der Form von Knochenschrauben (12); und
zwei mechanische Konstruktionen in der Form von Knochenschraubenaufnahmelöchern (14) entsprechend den beiden Knochenschrauben (12),
wobei jede Knochenschraube (12) einen Kopfabschnitt, der größer als das entsprechende Knochenschraubenaufnahmeloch (14) ist, und einen Knochenbefestigungsabschnitt aufweist, der kleiner als das entsprechende Knochenschraubenaufnahmeloch (14) ist; und
wobei die Abdeckung (31) dergestalt ist, dass, wenn sie gedreht ist, die Abdeckung (31) gleichzeitig beide Knochenschraubenkopfabschnitte überlappt, wenn sie in ihre entsprechenden Knochenschraubenaufnahmelöcher (14) eingesetzt sind, um **dadurch** ein Lösen der Knochenschrauben (12) zu verhindern.

12. Wirbelsäulenorthopädische Vorrichtung nach einem der Ansprüche 1 bis 11, wobei die Platte (50) aus einem ersten Material geformt ist, und der Ansatz (33) des Sicherungsmechanismus aus einem zweiten Material geformt ist.

13. Wirbelsäulenorthopädische Vorrichtung nach einem der Ansprüche 1 bis 12, wobei das Ansatzaufnahmeloch (55) eine Gestalt hat, die nichtkreisförmig ist, und der Ansatz (33) dieselbe Form wie das Ansatzaufnahmeloch (55) hat.

14. Wirbelsäulenorthopädische Vorrichtung nach Anspruch 13, wobei die Form oval ist.

15. Zwischenwirbelverbindungsprothese, aufweisend:
ein Kugelelement (22) für den Eingriff mit einem ersten Wirbel (V1), wobei das Kugelelement (22) eine im Allgemeinen konvexe Fläche (25) und eine erste Flanschplatte (26) an dem einen Ende des Kugelelements (22) für den Eingriff mit dem ersten Wirbel (V1) aufweist, wobei die erste Flanschplatte (26) eine erste Sicherungsmechanismusöffnung für den Eingriff mit einem ersten Sicherungsmechanismus und eine erste Knochenbefestigungsöffnung (29) für den Eingriff mit einem ersten Knochenbefestigungsmechanismus (12) aufweist;
ein Muldenelement (24) für den Eingriff mit einem zweiten Wirbel (V2) benachbart zu dem ersten Wirbel (V1), wobei das Muldenelement (24) eine im Allgemeinen konkave Fläche (27) und eine zweite Flanschplatte (28) an dem einen Ende des Muldenelements (24) für den Eingriff mit dem zweiten Wirbel (V2) aufweist, wobei die zweite Flanschplatte (28) eine zweite Sicherungsmechanismusöffnung für den Eingriff mit einem zweiten Sicherungsmechanismus und eine zweite Knochenbefestigungsöffnung (29) für den Eingriff mit einem zweiten Knochenbefestigungsmechanismus (12) aufweist; und
wobei der erste und zweite Sicherungsmechanismus für den Eingriff mit der ersten (26) bzw. der zweiten (28) Flanschplatte **dadurch gekennzeichnet sind, dass** jeder der Sicherungsmechanismen einen Keil (30) mit einer Abdeckung (31), einem Zapfen (32) und einem Ansatz (33) aufweist, wobei der Zapfen (32) ein erstes Ende (37), das mit der Abdeckung (31) verbunden ist, und ein zweites Ende (38) aufweist, das mit dem Ansatz verbunden ist, wobei sich wenigstens ein Teil des Ansatzes (33) von dem Durchmesser des zweiten Endes (38) des Zapfens (32) weg erstreckt; und
wobei jede der Flanschplatten (26, 28) ein nichtkreisförmiges Ansatzaufnahmeloch (55), eine mechanische Konstruktion für den Eingriff mit wenigstens einem Befestigungsmechanismus an einem Wirbel (V1, V2), und eine untere Ausnehmung an einer unteren Seite der Flanschplatte (26, 28) aufweist, wobei die untere Ausnehmung derart gestaltet ist, dass sie den Ansatz (33) aufnimmt und sichert, sobald der Keil (30) gedreht wird, wobei der Keil (30) und die Flanschplatte (26, 28) derart konfiguriert sind, dass der Ansatz durch das Ansatzaufnahmeloch (55) hindurch einsetzbar ist und drehbar ist, wenn er in dem Ansatzaufnahmeloch (55) eingesetzt ist, und wobei die Abdeckung (31) dergestalt ist, dass, wenn sie gedreht ist, die Abdeckung (31) wenigstens einen Teil der mechanischen Konstruktion überlappt, um **dadurch** ein Lösen des Befestigungsmechanismus zu verhindern, und
wobei die untere Ausnehmung ermöglicht, dass der Ansatz (33) in dieser aufgenommen wird, wenn der Keil (30) von einer Einsetzposition in eine Überlappungsposition derart gedreht ist, dass sich der Ansatz (33) nicht über die untere Fläche der Flanschplatte (26, 28) hinaus erstreckt.

16. Zwischenwirbelverbindungsprothese nach Anspruch 15, wobei die erste Flanschplatte (26) zwei Knochenbefestigungsmechanismusöffnungen (29) für den Eingriff mit zwei verschiedenen Knochenbefestigungsmechanismen (12) aufweist, und wobei die erste Sicherungsmechanismusabdeckung (30) derart konfiguriert ist, dass sie beide der Knochenbefestigungsmechanismen (12) der ersten Flanschplatte (26) sichert.

17. Zwischenwirbelverbindungsprothese nach Anspruch 15 oder 16, wobei die erste Flanschplatte (26) eine Aushöhlung (56) aufweist, um eine weitere Drehung des ersten Sicherungsmechanismus zu verhindern, nachdem der erste Sicherungsmechanismus in eine Position gedreht wurde, in welcher die Abdeckung (30) den ersten Knochenbefestigungsmechanismus (12) an der Stelle sichert.

## Revendications

1. Dispositif orthopédique rachidien comprenant :
une clavette (30) dotée d'un couvercle (31), d'un tenon (32) et d'un talon (33), le tenon (32) ayant une première extrémité (37) raccordée au couvercle (31) et une seconde extrémité (38) raccordée au talon (33) ; et au moins une partie du talon (33) qui s'étend hors du diamètre de la seconde extrémité (38) du tenon (32),
une plaque (50) ayant un orifice non circulaire (55) recevant le talon, un montage mécanique pour venir en prise avec au moins un mécanisme de fixation sur une vertèbre, la partie inférieure de la plaque (50) étant conçue pour recevoir et fixer le talon (33) une fois la rotation de la clavette (30) effectuée, la clavette (30) et la plaque (50) étant configurées de manière à ce que le talon puisse être inséré dans l'orifice (55) recevant le talon et puisse être tourné lorsqu'il est inséré dans l'orifice (55) recevant le talon, et
le couvercle (31) étant formé de manière à ce que, lorsqu'il est tourné, le couvercle (31) chevauche au moins une partie du montage mécanique pour empêcher de cette manière toute désolidarisation du mécanisme de fixation,
**caractérisé en ce que** la partie inférieure de la plaque comprend un évidement inférieur et **en ce que** l'évidement inférieur permet au talon (33) d'être contenu à l'intérieur de celui-ci lorsque la clavette (30) est tournée pour passer d'une position d'insertion à une position de chevauchement de manière à ce que le talon (33) ne s'étende pas au-delà de la surface inférieure de la plaque (50).

2. Dispositif orthopédique rachidien selon la revendication 1, dans lequel lé au moins un mécanisme de fixation est une vis à os (12) destinée à venir en prise avec un corps vertébral (V1, V2) et le montage mécanique est un orifice (29) recevant la vis à os.

3. Dispositif orthopédique rachidien selon la revendication 1 ou 2, dans lequel la plaque (50) a une partie supérieure (53) et une partie inférieure (54), la partie inférieure (54) ayant un logement (56) à proximité de l'orifice (55) recevant le talon pour permettre au talon (33) de venir en prise par rotation avec le logement (56).

4. Dispositif orthopédique rachidien selon l'une des revendications 1 à 3, dans lequel la plaque (50) comprend une paroi qui forme l'orifice (55) recevant le talon et un logement (56) est formé en tant que partie de la paroi, le tenon (32) ayant une forme qui vient en prise par rotation avec le logement (56).

5. Dispositif orthopédique rachidien selon la revendication 4, dans lequel le tenon (32) a une forme non circulaire.

6. Dispositif orthopédique rachidien selon l'une des revendications 1 à 5, dans lequel le talon (33) et l'orifice (55) recevant le talon sont non circulaires, de manière à ce qu'après l'insertion de la clavette (30) dans l'orifice (55) recevant le talon, la rotation de la clavette (30) par rapport à la plaque (50) positionne le talon (33) de manière à ce que le talon (33) empêche la clavette (30) de se retirer de l'orifice (55) recevant le talon.

7. Dispositif orthopédique rachidien selon l'une des revendications 1 à 6, dans lequel la plaque (50) comprend un évidement (15) et le couvercle (31) vient en prise de manière encastrée avec la plaque à l'intérieur de l'évidement (15).

8. Dispositif orthopédique rachidien selon la revendication 7, dans lequel le couvercle (31) a une surface supérieure (34) et une surface inférieure (35), de manière à ce que lorsque la surface inférieure (35) vient en prise avec la plaque (50), il y ait une transition régulière entre la plaque (50) et la surface supérieure (34) du couvercle.

9. Dispositif orthopédique rachidien selon l'une des revendications 1 à 8, dans lequel le couvercle (31) comprend une partie (60, 61) facilitant la rotation.

10. Dispositif orthopédique rachidien selon l'une des revendications 1 à 9 ; dans lequel la plaque (50) est dimensionnée pour réunir deux corps vertébraux (V1, V2) ou plus.

11. Dispositif orthopédique rachidien selon la revendication 1, comprenant :
deux mécanismes de fixation sous la forme de vis à os (12) ; et
deux montages mécaniques sous la forme d'orifices (14) recevant les vis à os correspondant aux deux vis à os (12),
dans lequel chaque vis à os (12) comprend une partie de tête qui est plus large que l'orifice (14) recevant la vis à os correspondante et une partie de fixation sur l'os qui est plus petite que l'orifice (14) recevant la vis à os correspondante ; et
dans lequel le couvercle (31) est formé de manière à ce que, lorsqu'il tourne, le couvercle (31) chevauche simultanément les deux parties de tête des vis à os lorsqu'elles sont insérées dans leurs orifices (14) recevant les vis à os correspondants pour empêcher de cette manière toute désolidarisation des vis à os (12).

12. Dispositif rachidien selon l'une des revendications 1 à 11, dans lequel la plaque (50) est formée à partir d'un premier matériau et le talon (33) du mécanisme de blocage est formé à partir d'un second matériau.

13. Dispositif rachidien selon l'une des revendications 1 à 12, dans lequel l'orifice (55) recevant le talon a une forme qui est non circulaire et le talon (33) a la même forme que l'orifice (55) recevant le talon.

14. Dispositif rachidien selon la revendication 13, dans lequel la forme est ovale.

15. Prothèse articulaire intervertébrale, comprenant :
un composant à boule (22) destiné à venir en prise avec une première vertèbre (V1), ledit composant à boule (22) comprenant une surface (25) généralement convexe et une première plaque d'appui (26) à une extrémité dudit composant à boule (22) pour venir en prise avec la première vertèbre (V1), la première plaque d'appui (26) comprenant une première ouverture pour mécanisme de blocage destinée à venir en prise avec un premier mécanisme de blocage et une première ouverture (29) de fixation sur l'os destinée à venir en prise avec un premier mécanisme (12) de fixation sur l'os ;
un composant creux (24) destiné à venir en prise avec une seconde vertèbre (V2) adjacente à la première vertèbre (V1), ledit composant creux (24) comprenant une surface (27) généralement concave et une seconde plaque d'appui (28) au niveau d'une extrémité dudit composant creux (24) destinée à venir en prise avec la seconde vertèbre (V2), la seconde plaque d'appui (28) comprenant une seconde ouverture pour mécanisme de blocage destinée à venir en prise avec un second mécanisme de blocage et une seconde ouverture (29) de fixation sur l'os destinée à venir en prise avec un second mécanisme (12) de fixation sur l'os ; et
les premier et second mécanismes de blocage pour venir en prise avec les première (26) et seconde (28) plaques d'appui, respectivement, **caractérisée en ce que** chacun desdits mécanismes de blocage comprend une clavette (30) ayant un couvercle (31), un tenon (32) et un talon (33), le tenon (32) ayant une première extrémité (37) raccordée au couvercle (31) et une seconde extrémité (38) raccordée au talon, au moins une partie du talon (33) s'étendant hors du diamètre de la seconde extrémité (38) du tenon (32) ; et
chacune desdites plaques d'appui (26, 28) ayant un orifice non circulaire (55) recevant le talon, un montage mécanique pour venir en prise avec au moins un mécanisme de fixation sur une vertèbre (V1, V2) et un évidement inférieur sur un côté inférieur de la plaque d'appui (26, 28), l'évidement inférieur étant conçu pour recevoir et fixer le talon (33) une fois que la clavette (30) a été tournée, la clavette (30) et la plaque d'appui (26, 28) étant configurées de manière à ce que le talon puisse être inséré dans l'orifice (55) recevant le talon et puisse être tourné lorsqu'il est inséré dans l'orifice (55) recevant le talon et le couvercle (31) est formé de manière à ce que lorsqu'il est tourné, le couvercle (31) chevauche au moins une partie du montage mécanique pour empêcher de cette manière toute désolidarisation du mécanisme de fixation, et
l'évidement inférieur permet au talon (33) d'être contenu à l'intérieur de celui-ci lorsque la clavette (30) est tournée pour passer d'une position d'insertion à une position de chevauchement de manière à ce que le talon (33) ne s'étende pas au-delà de la surface inférieure de la plaque d'appui (26, 28).

16. Prothèse articulaire intervertébrale selon la revendication 15, dans laquelle la première plaque d'appui (626) comprend deux ouvertures (29) pour mécanisme de fixation sur l'os destinées à venir en prise avec deux mécanismes de fixation (12) sur l'os différents et dans laquelle le couvercle (30) du premier mécanisme de blocage est configuré pour fixer les deux mécanismes de fixation (12) sur l'os de la première plaque d'appui (26).

17. Prothèse articulaire intervertébrale selon la revendication 15 ou 16 dans laquelle la première plaque d'appui (26) comprend un logement (56) pour empêcher le premier mécanisme de blocage de tourner davantage après que le premier mécanisme de blocage a été tourné jusqu'à atteindre une position dans laquelle le couvercle (30) fixe en place le premier mécanisme de fixation (12).
